(19) **Europäisches Patentamt**
**European Patent Office**
**Office européen des brevets**

(11) **EP 1 490 026 B1**

(12) **EUROPÄISCHE PATENTSCHRIFT**

(45) Veröffentlichungstag und Bekanntmachung des Hinweises auf die Patenterteilung:
**01.12.2010 Patentblatt 2010/48**

(21) Anmeldenummer: **03727164.0**

(22) Anmeldetag: **01.04.2003**

(51) Int Cl.:
**A61K 9/127** *(2006.01)*    A61K 9/72 *(2006.01)*

(86) Internationale Anmeldenummer:
**PCT/DE2003/001068**

(87) Internationale Veröffentlichungsnummer:
**WO 2003/084507 (16.10.2003 Gazette 2003/42)**

(54) **VERNEBELBARE LIPOSOMEN UND IHRE VERWENDUNG ZUR PULMONALEN APPLIKATION VON WIRKSTOFFEN**

ATOMIZABLE LIPOSOMES AND THEIR USE FOR THE PULMONARY ADMINISTRATION OF ACTIVE SUBSTANCES

LIPOSOMES ATOMISABLES ET UTILISATION POUR L'APPLICATION PULMONAIRE D'AGENTS ACTIFS

(84) Benannte Vertragsstaaten:
**AT BE BG CH CY CZ DE DK EE ES FI FR GB GR HU IE IT LI LU MC NL PT RO SE SI SK TR**

(30) Priorität: **04.04.2002 DE 10214983**

(43) Veröffentlichungstag der Anmeldung:
**29.12.2004 Patentblatt 2004/53**

(73) Patentinhaber: **United Therapeutics Corporation Silver Spring, MD 20910 (US)**

(72) Erfinder:
• **SCHMEHL, Thomas**
**35390 Giessen (DE)**
• **GESSLER, Tobias**
**35435 Wettenberg (DE)**
• **WASCHKOWITZ, Esther**
**35392 Giessen (DE)**

(74) Vertreter: **Buchhold, Jürgen**
**Patentanwälte Olbricht & Buchhold**
**Am Weinberg 15**
**35096 Weimar/Lahn (DE)**

(56) Entgegenhaltungen:
**EP-A- 0 361 894    WO-A-02/03959**
**US-A- 5 662 929**

• **SINGH M ET AL: "Stealth monensin liposomes as a potentiator of adriamycin in cancer treatment" JOURNAL OF CONTROLLED RELEASE, ELSEVIER SCIENCE PUBLISHERS B.V., AMSTERDAM, NL, Bd. 59, Nr. 1, 1. Mai 1999 (1999-05-01), Seiten 43-53, XP004166214 ISSN: 0168-3659**

EP 1 490 026 B1

**Beschreibung**

Die vorliegende Erfindung betrifft die Herstellung von Liposomen, die stabil gegenüber Vernebelung und zur pulmonalen Applikation geeignet sind und die eine verzögerte Freisetzung von eingeschlossenen Wirkstoffen zeigen.

In der pharmazeutischen Technologie unterscheidet man verschiedene Arzneiformen mit protrahierter Wirkung. Sie alle haben das Ziel, eine Wirkungsverlängerung des jeweiligen Arzneistoffes zu erreichen. Man unterscheidet Langzeit-, Depot- und Retardpräparate. Die modifizierte Wirkstofffreigabe kann durch chemische Veränderungen (z.B. Salze, Ester, Komplexe), durch Zusätze (z.B. Verwendung von Hilfsstoffen), durch technologische Maßnahmen (z.B. Wahl bestimmter Korngrößen, Tablettenhärten) sowie durch Wahl des Applikationsortes oder der Applikationsart erreicht werden. In Abhängigkeit von der Art der Freigabe unterscheidet man den sustained-, repeatprolonged-, und delayed-release-Typ.

Ein besonderes Problem stellt die Applikation von pharmazeutischen Wirkstoffen in der Lunge dar, da hier aus Verträglichkeitsgründen die Auswahl der Hilfsstoffe stark eingeschränkt und eine gezielte und kontrollierte Freisetzung schwierig zu erreichen ist.

Als toxikologisch unbedenklich gelten Liposomen, wie die bereits für andere Applikationswege zugelassenen liposomalen Produkte Daunoxome®, Ambisome®, HeparinPur® u.a. zeigen. Liposomale Formulierungen erscheinen auch für die Lunge als geeignet, da sie ganz oder zum größten Teil aus körpereigenen Substanzen hergestellt werden können, die natürlicherweise in der Lunge vorkommen.

Als Liposomen bezeichnet der Fachmann Lipidvesikel, die in ihrer Struktur körpereigenen Zellmembranen stark ähneln. Die Vesikelwände, die beispielsweise aus Phospholipiden bestehen, trennen einen wässrigen Innenraum von der ebenfalls wässrigen umgebenden Phase. Derartige Lipidvesikel können sich spontan nach thermodynamischen Gesetzmäßigkeiten u.a. durch Dispersion von Phospholipiden in überschüssigem wässrigen Medium bilden. Zur Herstellung von Liposomen werden üblicherweise natürliche, partialsynthetische oder auch vollsynthetische Phospholipide eingesetzt. Während andere Lipide einen kegelförmigen Molekülaufbau zeigen, der die Bildung von Mizellen favorisiert, weisen Phospholipide aufgrund der beiden Fettsäureketten eine zylindrische Geometrie auf, die in wässrigem Medium die Bildung von lamellaren Strukturen begünstigt.

Der Aufbau der einzelnen Lamellen eines Liposoms ist ähnlich dem der Zellmembran: Die Phospholipide bilden Doppelschichten, in denen sich die hydrophoben Molekülteile im Inneren der Doppelschicht befinden, während die hydrophilen Kopfgruppen in die wässrige Phase nach außen beziehungsweise innen zeigen.

Aufgrund ihrer Struktur und Größe lassen sich die Liposomen in verschiedene Gruppen einteilen (Tabelle 1).

Tabelle 1

| Klassifizierung | Durchmesser | Struktur |
|---|---|---|
| MLV (multilamellar vesicles) | 100-5000 nm | mehrere Doppelmembranen |
| SUV (small unilamellar vesicles) | < 100 nm | unilamellare, kleine, hydrophil gefüllte Vesikel |
| LUV (large unilamellar vesicles) | > 100 nm (bis 2000 nm) | unilamellare, große, hydrophil gefüllte Vesikel |

Da Liposomen sowohl hydrophile als auch lipophile Bereiche aufweisen, können Wirkstoffe unterschiedlich eingebaut werden. Während sich lipophile Wirkstoffe in die Membran einlagern, befinden sich hydrophile Stoffe in den wässrigen Schichten der Vesikelwände oder im von den Vesikelwänden begrenzten wässrigen Innenraum der Liposomen. Amphiphile Wirkstoffe ordnen sich analog den Phospholipiden an. Die unterschiedlichen Eigenschaften der Liposomenklassen bestimmen die möglichen Einsatzgebiete. So weisen SUV im Verhältnis einen hohen Anteil an Lipiden auf und eignen sich von daher vor allem für lipophile Wirkstoffe; für LUV gilt das Gegenteil, sie weisen einen verhältnismäßig großen Innenraum zur Aufnahme hydrophiler Wirkstoffe auf. Eine verzögerte Freisetzung ist am ehesten durch eine MLV-Struktur mit den vielen hintereinander geschalteten Diffusionsmembranen realisierbar.

Sowohl durch ihre biologische Verträglichkeit als auch durch die Möglichkeit einer modifizierbaren Pharmakokinetik (z.B. verzögerte Wirkstofffreisetzung) stellen Liposomen einen vielversprechenden Ansatz für eine kontrollierte und verzögerte Freisetzung von Wirkstoffen in der Lunge dar.

Eine derartige kontrollierte und verzögerte Freisetzung von Wirkstoffen in der Lunge könnte für eine Vielzahl von Erkrankungen von erheblicher therapeutischer Bedeutung sein. Insbesondere bietet sich bei Lungenerkrankungen die Möglichkeit, Wirkstoffe direkt in das Zielorgan zu verbringen und dort eine hohe lokale Wirkstoffkonzentration bei langer Wirkdauer zu erzielen. Die retardierte Wirkstofffreisetzung kann darüber hinaus zu einer Verminderung von Nebenwirkungen führen, da zum einen starke Konzentrationsschwankungen mit hohen Konzentrations-Peaks unmittelbar nach der Applikation vermieden werden, zum anderen durch die liposomale Verpackung Wirkstoffe mit kurzer biologischer Halbwertszeit Verwendung finden können, die nach Freisetzung und Wirkungsvermittlung schnell inaktiviert werden. In Gegensatz dazu können Wirkstoffe mit langer biologischer Halbwertszeit, wie sie teilweise zur Behandlung des Asthmas oder chronisch obstruktiver Lungenerkrankungen (COPD) eingesetzt werden, über die Luft-Blut-Barriere in den Körperkreislauf gelangen und dort zu Nebenwirkungen führen. Neben den bereits erwähnten Lungen- und Atemwegserkrankungen Asthma und COPD stellen insbesondere die Lungenentzündung und der Lungenhochdruck Erkrankungen dar, die durch liposomal verpackte Wirkstoffe besser behandelt werden könnten. So sind beispielsweise derzeit in der Therapie des Lungenhochdrucks mit gefäßaktiven Substanzen (z.B. Prostazyklin oder Prostazyklinanaloga) aufgrund deren kurzen biologischen Halbwertszeiten häufige, zum Teil stündliche Inhalationen mit einer Dauer von bis zu 15 Minuten für eine effektive und anhaltende Drucksenkung im Lungenkreislauf notwendig. Liposomale Verpackung und retardierte Freisetzung der Substanzen könnten die Inhalationshäufigkeit zum Wohle der Patienten drastisch reduzieren und gleichzeitig zu einer konstanten, auch über die Nachtphase anhaltenden, therapeutisch erwünschten Drucksenkung im Lungenkreislauf sorgen.

Neben der Anwendung bei Lungenerkrankungen ist die verzögerte und kontrollierte Freisetzung von Wirkstoffen auch für systemische Erkrankungen, wie z.B. Diabetes mellitus, von Interesse. Die Lunge ist ein Organ, das aufgrund der äußerst dünnen Grenzschicht zwischen Luft und Blut und der großen Gesamtfläche der Lungenbläschen über ein hohes Resorptionsvermögen verfügt. Daher können transpulmonal applizierte Wirkstoffe systemisch verfügbar gemacht und entsprechend zur Prophylaxe oder Therapie von systemischen Erkrankungen eingesetzt werden. Beispielsweise wird derzeit die inhalative Bolusgabe von Insulin als Alternative zur Injektion entwickelt, wobei jedoch bisher keine Formulierung zur kontinuierlichen basalen Insulinfreisetzung in den Körperkreislauf bekannt ist. Bislang gibt es keine Depotform zur pulmonalen Applikation. Die bisher einzige Möglichkeit für eine lange Wirkdauer besteht in der Verwendung von Arzneistoffen mit entsprechend langer Halbwertszeit.

Das Standardverfahren zum Einbringen von Wirkstoffen in die Lunge ist die Inhalation von Aerosolen. Wirkstofftragende Aerosole werden mittels geeigneter Aerosolgeneratoren erzeugt, in der Praxis übliche Geräte sind Düsen- und Ultraschallverneblung sowie Dosieraerosole und Pulverinhalatoren. Die Deposition von Aerosolen im Atemtrakt ist insbesondere abhängig von der Partikelgrößenverteilung des Aerosols. Partikel mit einem aerodynamischen Massendurchmesser von unter 6 μm erreichen zu einem größeren Prozentsatz die Lunge mit Luftröhre, Bronchien und Alveolarbereich; zu therapeutischen Zwecken sind entsprechend Aerosole mit Partikeldurchmessern kleiner 6 μm zu verwenden.

Wirkstoff-Formulierungen, die in wässriger Lösung vorliegen, lassen sich mit Düsen- oder Ultraschall-Aerosolgeneratoren vernebeln. Für die Anwendung mit Dosieraerosolen oder Pulverinhalatoren sind zusätzliche Modifikationen der Wirkstoff-Formulierungen notwendig (z.B. Lösung oder Suspension in Treibmitteln, Mikronisierung als Trockenpulver). Bei wässrigen liposomalen Dispersionen bietet sich in besonderer Weise die Düsen- und Ultraschallverneblung als Verfahren zur Aerosolerzeugung an. Beiden Verfahren gemeinsam ist, dass durch Einwirkung mechanischer Energie Aerosoltröpfchen aus dem Flüssigkeitskontinuum herausgelöst werden. Bei diesen Prozessen wirken Kräfte auf die zu vernebelnde Flüssigkeit und auf die suspendierten Lipidvesikel, die zu einer Beeinträchtigung der Integrität der Lipidvesikel und damit zu einer vorzeitigen Freisetzung des in die Vesikel eingeschlossenen Wirkstoffs führen können. Eine Grundvoraussetzung für die pulmonale Anwendbarkeit von liposomalen Depotformulierungen als Aerosol ist daher eine ausreichende Stabilität der Liposomen gegenüber dem Verneblungsprozess. Die Stabilität von Liposomen gegenüber der Verneblung hängt neben den technischen Bedingungen der verschiedenen Verneblungsprozesse (Druck, Ultraschallfrequenz) insbesondere von der Liposomenart und Liposomengröße sowie von der chemischen Zusammensetzung der Liposomenmembranen ab. Eine weitere Möglichkeit zum Einbringen von Wirkstoffen in die Lunge ist die intratracheale Instillation. Bei dieser Methode wird ein Schlauch in die Luftröhre oder in die tieferliegenden Bronchien eingeführt und die Wirkstofflösung über diesen in den Atemtrakt verbracht. Dieses Verfahren stellt zwar wesentlich geringere Anforderungen an die Stabilität von liposomalen Formulierungen als die Verneblung, führt jedoch zu einer inhomogenen Verteilung des eingebrachten Materials in der Lunge. Darüber hinaus ist die intratracheale Instillation aufgrund ihrer Invasivität nur äußerst begrenzt einsetzbar und eignet sich nicht für eine Heim- oder Dauertherapie. Um als pulmonale Depotform geeignet zu sein, muss eine liposomale Formulierung eine ausreichende Stabilität gegenüber dem Vernebelungsprozess besitzen, mittels Vernebelungstechniken in ein lungengängiges Aerosol überführt werden können, und eine verzögerte Wirkstofffreisetzung am Zielort aufweisen. Idealerweise beginnt die Freisetzung des Wirkstoffes direkt nach der Deposition der Liposomen in der Lunge und hält über mehrere Stunden an. EP 0 361 894 offenbart die Herstellung stabiler Liposomer mit verzögerter Wirkstofffreisetzung. Aufgabe der vorliegenden Erfindung ist es, eine spezifische, nicht toxische Formulierung zur pulmonalen Applikation bereitzustellen, die vernebelbar und lungengängig ist und eine verzögerte Freisetzung von eingeschlossenen Wirkstoffen und/oder Farbstoffen nach der Deposition aufweist.

Diese Aufgabe wird erfindungsgemäß durch die Bereitstellung von Formulierungen gemäß Anspruch 1 gelöst.

Die liposomalen Formulierungen werden nach der dem Fachmann bekannten Filmmethode hergestellt (siehe beispielsweise das Lehrbuch von R. R. C. New (Editor), Liposomes: a practical approach, Oxford University Press, Reprint 1994). Als weiterer Arbeitsschritt erfolgt die Extrusion der Liposomen durch Filtermembranen sowie das Abtrennen des nichtverkapselten, freien Wirkstoffanteils durch Zentrifugation, Dialyse oder chromatographische Verfahren.

Zur Bestimmung der Effizienz der Einschlußreaktion können die Liposomen mit Methanol zerstört und anschließend die Konzentration des Wirkstoffs mittels geeigneter Methoden gemessen werden. Zur Charakterisierung der Stabilität der Liposomen gegenüber der Verneblung wird die Liposomendispersion mittels gängiger Aerosolgeneratoren vernebelt, das entstehende Aerosol aufgefangen und anschließend der freie und der verkapselte Anteil des Wirkstoffs bestimmt.

Die Liposomen sind überraschenderweise gut mit gängigen Düsen- und Ultraschallverneblern aerosolierbar. Beim Vernebeln der liposomalen Dispersionen findet man vergleichbare Partikelgrößenverteilungen des entstehenden Aerosols wie beim Vernebeln von isotonischer Kochsalzlösung, so dass mit geeigneten handelsüblichen Verneblern lungengängige Aerosole aus den Liposomendispersionen erzeugt werden können. (Beispiele für handelsübliche Vernebler: Düsenvernebler wie Bennett-Raindrop®, Pari LC®, Pari LL®, Ventstream®, Ultraschallvernebler wie Multisonic pro®, Pulmosonic®, Systam LS®).

**Ausführungsbeispiele:**

Tabelle 2 zeigt die erfindungsgemäßen liposomalen Formulierungen, die aus den natürlichen Bestandteilen des Lungensurfactant Dipalmitoylphosphatidylcholin (DPPC), Cholesterin (Chol), Dimyristoylphosphatidylcholin (DMPC), Sphingomyelin (SM) und dem nicht im Lungensurfactant vorkommenden Polyethylenglykol (PEG) kombiniert werden.

Tabelle 2

| Liposomenformulierungen | (DPPC) | (Chol) | (PEG) | (DMPC) | (SM) |
|---|---|---|---|---|---|
| **Ausführungsbeispiel 1** | 7 | 3 | 0,15 | - | - |
| | 7 | 3 | 0,3 | - | - |
| | 7 | 3 | 0,6 | - | - |
| **Ausführungsbeispiel 2** | 7 | 4 | - | 1 | - |
| | 7 | 4 | - | 2 | - |
| | 7 | | - | 3 | - |
| | 7 | 4 | - | 4 | - |
| **Ausführungsbeispiel 3** | 7 | 3 | - | - | 2% |
| | 7 | 3 | - | - | 4% |
| | 7 | 3 | - | - | 6% |
| | 7 | 3 | - | - | 8% |

Die Zahlen geben das molare Verhältnis der Komponenten DPPC, Chol, PEG und DMPC an; bei SM sind Gewichtsprozent angegeben.

**Liposomenherstellung**

Mit den erfindungsgemäßen liposomalen Formulierungen können hydrophile, lipophile und/oder amphiphile Wirk- oder Farbstoffe mit ausreichender Stabilität gegenüber der Verneblung verkapselt werden.

In den Ausführungsbeispielen wird als Modellwirkstoff Carboxyfluorescein verwendet, ein hydrophiler Farbstoff, der sich einfach mittels Fluoreszenzspektroskopie nachweisen läßt. Zur Herstellung der Carboxyfluorescein-Lösung (CF-Lösung) werden 100 mg 5-(6)-Carboxyfluorescein in 10 ml PBS-Puffer gelöst und der pH-Wert der CF-Lösung auf 7,4 eingestellt. Zur Liposomenherstellung nach der Filmmethode wird 150 mg Gesamtmenge an Phospholipiden (siehe Formulierungen Tabelle 2) in 40 ml eines Gemisches aus Chloroform und Methanol (70:30 Volumenverhältnis) in einem Rundkolben gelöst und das Lösungsmittel bei 60°C im Rotationsverdampfer unter Vakuum abgezogen. Der an der Innenwand des Kolbens entstehende dünne Film wird weiter 2h unter Vakuum getrocknet. Durch Zugabe von 10 ml der ebenfalls auf 60°C erwärmten CF-Lösung erfolgt die Hydratisierung des Filmes. Die entstandene Dispersion wird für 2h unter Erwärmung gerührt und vor der Extrusion einem Frier-Tau-Zyklus unterzogen. Dazu wird der Rundkolben in flüssigen Stickstoff getaucht bis der Inhalt gefroren ist. Während des Auftauens bei Raumtemperatur reißen die Membranen auf, schließen zusätzliches CF ein und erhöhen dadurch die Effizienz der Verkapselung. Dieser Zyklus wird fünfmal wiederholt. Danach wird die Dispersion erneut im Wasserbad auf 60°C erwärmt. Jeweils 0,5 ml der Dispersion werden 21mal durch eine Polycarbonat-Filtermembran mit einem Porendurchmesser von 1,0 μm bzw. 0,4 μm extrudiert. Die Dispersion wird anschließend bei 4°C zentrifugiert (4 x 45 min/ 4500 rpm bzw. 4 x 60 min/ 15000 rpm). Die überstehende CF-Lösung wird jeweils nach den einzelnen Zentrifugationszyklen abpipettiert und durch ein gleiches Volumen an PBS-Puffer ersetzt.

**Ausführungsbeispiel 1**

Diese Liposomenformulierungen enthalten ein molares Verhältnis von Dipalmitoylphosphatidylcholin (DPPC), Cholesterin (Chol) und Polyethylenglykol (PEG) von 7:3:0,15 bis 7:3:0,6.

**Ausführungsbeispiel 2**

Diese Liposomenformulierungen enthalten ein molares Verhältnis von Dipalmitoylphosphatidylcholin (DPPC), Cholesterin (Chol) und Dimyristoylphosphatidylcholin (DMPC) von 7:4:1 bis 7:4:4.

**Ausführungsbeispiel 3**

Diese Liposomenformulierungen enthalten ein molares Verhältnis von Dipalmitoylphosphatidylcholin (DPPC) und Cholesterin (Chol) von 7:3 als 100 Gewichtsprozent, dazu variierende Mengen an Sphingomyelin (SM) von mehr als 2%, besonders bevorzugt 2-8% Gewichtsprozent.
Ebenfalls vorteilhafte Eigenschaften haben Liposomenformulierungen mit Dipalmitoylphosphatidylcholin (DPPC) und Cholesterin (Chol) und Kombinationen von DMPC, SM und/oder PEG.
Die erfindungsgemäßen Liposomen haben eine Größe von 0,2 - 1,5 $\mu$m.
Tabelle 3 zeigt exemplarisch die Größe der Liposomen eines Herstellungsganges nach Extrusion durch eine Polycarbonatmembran mit 0,4 $\mu$m Porengröße, Zentrifugation und Verneblung mit einem Düsenvernebler (dargestellt sind die Mittelwerte mit Standardabweichungen, n=3).

Tabelle 3

| Formulierungen | Liposomen durchmesser nach Extrusion [um] | Liposomen durchmesser nach Zentrifugation [$\mu$m] | Liposomen durchmesser nach Düsenverneblung [$\mu$m] |
|---|---|---|---|
| DPPC:Chol:DMPC 7:4:1 7:4:2 7:4:3 7:4:4 | 0,57 +/- 0,01 | 0,58 +/- 0,01 | 0,51 +/- 0,01 |
| DPPC:Chol:PEG 7:3:0,15 7:3:0,3 7:3:0,6 | 0,60 +/- 0,02 | 0,57 +/- 0,01 | 0,64 +/- 0,01 |
| DPPC:Chol:SM 7:3 mit 2% SM 7:3 mit 4% SM 7:3 mit 6% SM 7:3 mit 8% SM | 0,65 +/- 0,01 | 0,68 +/- 0,02 | 0,70 +/- 0,01 |

**Stabilität der Liposomen gegenüber Vernebelung**

Zur Untersuchung der Stabilität gegenüber Vernebelung werden die durch eine 0,4 4 $\mu$m Polycarbonatfilter-membran extrudierten liposomalen Formulierungen mit PBS 1:10 verdünnt und jeweils 2,5 ml der erhaltenen Dispersion für 3 Minuten bei Raumtemperatur mit Düsen- (betrieben mit 1 bar Druckluft) und Ultraschallverneblern (betrieben mit 10 1/min Zusatzluft) vernebelt. Das erzeugte Aerosol wird über einen Silikonschlauch (15 cm lang, 3 cm Innendurchmesser) auf eine Prallplatte aus Glas geleitet und in einem 40 ml Reagenzgefäß aufgefangen. Der freie und der gesamte Anteil an Carboxyfluorescein in den aufgefangenen Dispersionen wird fluoreszenzspektrometrisch analysiert.
Zur Bestimmung des freien Anteils an CF ($c_{frei}$) werden 100 $\mu$l der erfindungsgemäßen liposomalen Dispersion in einem Eppendorfgefäß zentrifugiert, 50 $\mu$l des erhaltenen Überstandes mit PBS-Puffer 1:100 verdünnt und die CF-Konzentration im Überstand fluoreszenzspektrometrisch quantifiziert. Zur Bestimmung des gesamten Anteils an CF ($c_{gesamt}$) werden 50 $\mu$l der erfindungsgemäßen liposomalen Dispersion mit 450 $\mu$l Methanol versetzt, 10 min inkubiert und anschließend zentrifugiert. Die Zugabe von Methanol bewirkt dabei die Zerstörung der Liposomen und eine vollständigen Freisetzung des Farbstoffes. Nach der Zentrifugation werden 50 µl des Überstands mit PBS 1:1000 verdünnt und die Konzentration von CF fluoreszenzspektrometrisch quantifiziert. Der in den Liposomen verkapselte Anteil des Farbstoffes ($c_{verkapselt}$) läßt sich nun einfach anhand der folgenden Formel bestimmen:

$$c_{verkapselt} = (c_{gesamt} - c_{frei})*100 / c_{gesamt} \quad [\%]$$

Die Untersuchungen zur Stabilität der Liposomen gegenüber Verneblung zeigen, daß 50% - 80% der Liposomen den Verneblungsprozess mit Düsen- bzw. Ultraschallverneblern als intakte Liposomen überstehen.

Tabelle 4 zeigt exemplarisch die Stabilität der Liposomen gegenüber Düsenverneblung für ausgewählte Ausführungsbeispiele (Mittelwerte $\pm$ Standardabweichungen, n = 4).

Tabelle 4

| Formulierungen | Anteil an liposomal verpacktem Wirkstoff nach Verneblung [%] |
|---|---|
| DPPC:Chol:DMPC 7:4:1 7:4:2 7:4:3 7:4:4 | 69,3 $\pm$ 1,3 |
| DPPC:Chol:PEG 7:3:0,15 7:3:0,3 7:3:0,6 | 55,7 $\pm$ 1,8 |
| DPPC:Chol:SM 7:3 mit 2% SM 7:3 mit 4% SM 7:3 mit 6% SM 7:3 mit 8% SM | 76,9 $\pm$ 0,7 |

**Freisetzungskinetik der lipsosmal verpackten Wirk- und Farbstoffe am Lungenmodell**

Die erfindungsgemäßen liposomalen Dispersionen werden am Modell der isoliert perfundierten und ventilierten Kaninchenlunge (Seeger et al., J Appl Physiol 1986, 61:1781-1789; Seeger et al., In: Oxygen Radicals in Biological Systems, edited by L. Packer. New York: Academic Press, 1994, vol. 233:549-584) untersucht. Dabei werden Kaninchen mit einen Gewicht zwischen 2,5 und 3 kg mit Heparin antikoaguliert und mit einem Gemisch aus Xylocain, Ketamin, und Xylazin anaesthesiert. Unter Beatmung mit Raumluft erfolgt eine Tracheostomie. Nach mittiger sternaler Thorakotomie wird ein Katheter in die Pulmonalarterie eingebunden, die Aorta ligiert und die beiden Ventrikel an der Herzspitze eröffnet.

Die Perfusion der Lunge erfolgt nun mittels einer Rollerpumpe, als Perfusionsmedium wird Krebs-Henseleit-Elektrolytlösung verwendet, die zusätzlich Hydroxyethylstärke und Natrium-Bicarbonat enthält. Parallel zu der künstlichen Perfusion wird die Lunge mit einem Gemisch aus 21% Sauerstoff, 5,3% Kohlendioxid und 73,7% Stickstoff beatmet. Das Herz-Lungen-Paket wird nun vorsichtig aus der Brusthöhle präpariert und anschließend ein zweiter Katheter im linken Ventrikel mittels einer Tabaksbeutelnaht fixiert. Nach Aufhängen der Lunge an einem Gewichtsmesser wird der venöse Schenkel des Perfusionssystems mit dem in das Herz eingebundenen Katheter verbunden, so dass ein geschlosser, rezirkulierender Perfusionskreislauf entsteht. Zur Aufrechterhaltung einer physiologischen Temperatur wird das Perfusat auf 38°C geheizt und die frei hängende Lunge in eine beheizte Kammer verbracht.

Die Standardparameter der Lungenventilation und -perfusion sind:

- Die Lungenperfusion mittels Rollerpumpe beträgt 100 ml/min.
- Die Lungenventilation erfolgt mit einem Atemzugvolumen von 30 ml und einer Atemfrequenz von 30 $min^{-1}$.
- Der positive endexspiratorische Druck (PEEP) beträgt 1 mmHg.
- Der linksventrikuläre Druck wird auf 2 mmHg eingestellt.

- Das Perfusatvolumen beträgt 150 ml.

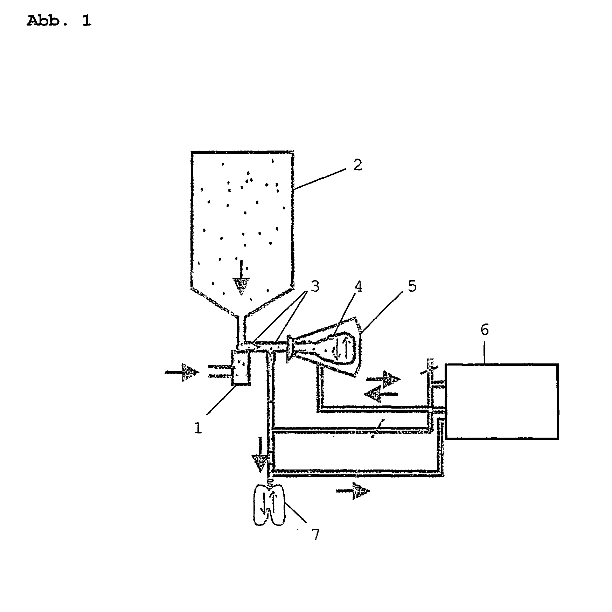

Die inhalative Applikation von Aerosolen erfolgt über ein sog. Bag-in-Box-System (Abb. 1). Dieses System besteht aus einem Düsenvernebler (1), Vorratsbeutel (2), Ventilen (3) und einem flexiblen Ballon (4, Bag), der in ein luftdichtes Glasgefäß (5, Box) eingebracht ist. Die Beatmungspumpe (6) ist an das luftdichte Glasgefäß (5) angeschlossen und vermittelte je nach Richtung des Gasflusses einen Über- oder Unterdruck auf den im Glasgefäß (5) befindlichen flexiblen Ballon (4). Das vom Düsenvernebler (1) erzeugte Aerosol wird zunächst in den Vorratsbeutel (2) geleitet. Während der Expirationsphase der Pumpe (6) wird der flexible Ballon (4) aufgrund des Unterdrucks im Glasgefäß (5) gedehnt und über ein Ventilsystem (3) mit Aerosol aus dem Vorratsbeutel gefüllt. In der darauffolgenden Inspirationsphase wird das Aerosol bei entsprechender Ventilstellung aus dem Ballon (4) in den Atemtrakt der Lunge (7) verbracht.

Zur Verneblung wird ein Düsenvernebler (Bennett-Raindrop®) verwendet, der mit Atemgasgemisch (5,3% $CO_2$, 21% $O_2$, 73,7% $N_2$) bei einem Druck von 1,0 bar betrieben wird.

Zur Bestimmung der Freisetzungskinetik von liposomal verkapseltem Farb- oder Wirkstoff werden in einer ersten Versuchsreihe an jeder Lunge zwei aufeinanderfolgende Interventionen durchgeführt. Zunächst erfolgt die inhalative Applikation einer definierten Menge an aerosolierter CF-Lösung über einen Zeitraum von 30 Minuten, gefolgt von einer Beobachtungszeit von 90 Minuten. Nach einem Perfusatwechsel mit 1 L frischer Elektrolytlösung wird in einem zweiten Schritt die jeweilige liposmale Dispersion ebenfalls über 30 aerosoliert, gefolgt von einer Beobachtungszeit von bis zu 270 Minuten. Während beider inhalativen Aerosolapplikationen wird die gleiche Menge an CF in der Kaninchenlunge deponiert. Freies CF tritt bekanntermaßen schnell und vollständig aus dem Alveolarraum in das Gefäßsystem der Lunge über. Durch Probenentnahmen (0,5 ml) aus dem Perfusat, die beginnend mit dem Zeitpunkt unmittelbar vor der Aerosolapplikation alle 10 Minuten erfolgen, kann der Übertritt des Farbstoff quantifiziert werden. Im Vergleich mit der unverkapselten CF-Lösung läßt sich nun die Freisetzungskinetik des in Liposomen verkapselten CF beurteilen.

Die Freisetzungskinetik des Modell-Wirkstoffs CF für die erfindungsgemäßen Formulierungen ist in den Abbildungen 2-4 dargestellt (Abb.2 Freisetzungskinetik von DPPC/Chol/DMPC-Liposomen, Abb.3 Freisetzungskinetik von DPPC/Chol/PEG-Liposomen, Abb.4 Freisetzungskinetik von DPPC/Chol/SM-Liposomen).

**Abb. 1 Darstellung des Bag-in-Box-Systems zur Beatmung der isolierten Lunge**

Die Beatmungspumpe (6) bewegt einen flexiblen Ballon (4), der aus einem Vorratsbeutel (2) die Lunge (7) mit dem Aerosol/Atemgasgemisch versorgt. Die Füllung des Vorratsbeutels (2) erfolgt mit Aerosol, das mittels eines Düsenverneblers (1) erzeugt wird, der durch Atemgas mit 1,0 bar betrieben wird.

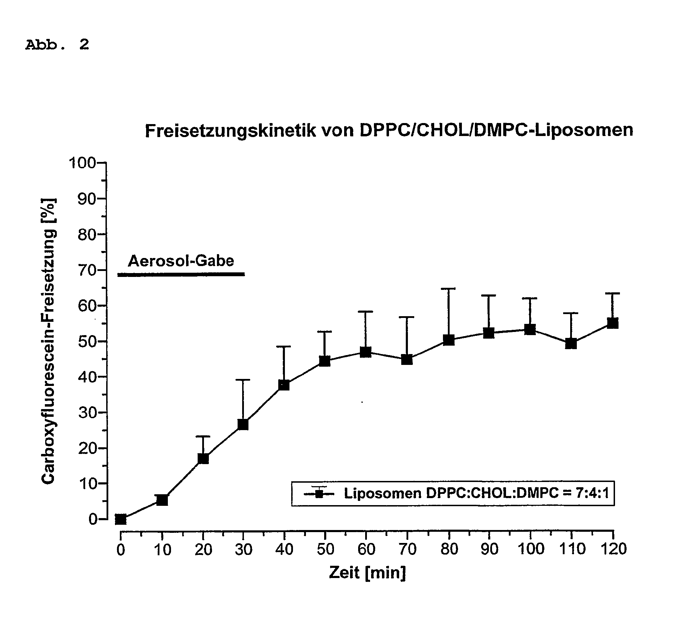

**Abb. 2 Freisetzungskinetik von DPPC/Chol/DMPC-Liposomen**

Liposomen der Zusammensetzung DPPC:Chol:DMPC = 7:4:1 mit Carboxyfluorescein als eingeschlossenem Modell-Wirkstoff werden über einen Zeitraum von 30 Minuten als Aerosol in eine isoliert ventilierte und perfundierte Kaninchenlunge eingebracht. Das aus den Liposomen freigesetzte und im Perfusionsmedium nachgewiesene Carboxyfluorescein ist in Prozent des gesamten initial in den Liposomen eingeschlossenen Carboxyfluoresceins angegeben. Dargestellt sind Mittelwerte mit Standardabweichung aus n = 3 Versuchen.

DPPC = Dipalmitoylphosphatidylcholin, Chol = Cholesterin DMPC = Dimyristoylphosphatidylcholin

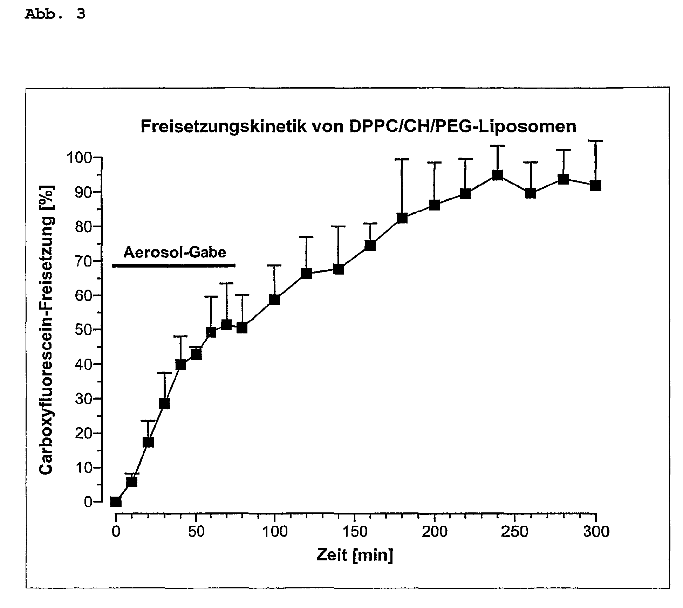

**Abb. 3 Freisetzungskinetik von DPPC/Chol/PEG-Liposomen**

Liposomen der Zusammensetzung DPPC:Chol:PEG = 7:3:0.3 mit Carboxyfluorescein als eingeschlossenem Modell-Wirkstoff werden über einen Zeitraum von 30 Minuten als Aerosol in eine isoliert ventilierte und perfundierte Kaninchenlunge eingebracht.

Das aus den Liposomen freigesetzte und im Perfusionsmedium nachgewiesene Carboxyfluorescein ist in Prozent des gesamten initial in den Liposomen eingeschlossenen Carboxyfluoresceins angegeben. Dargestellt sind Mittelwerte mit Standardabweichung aus n = 3 Versuchen.

DPPC = Dipalmitoylphosphatidylcholin, Chol = Cholesterin
PEG = Polyethylenglykol

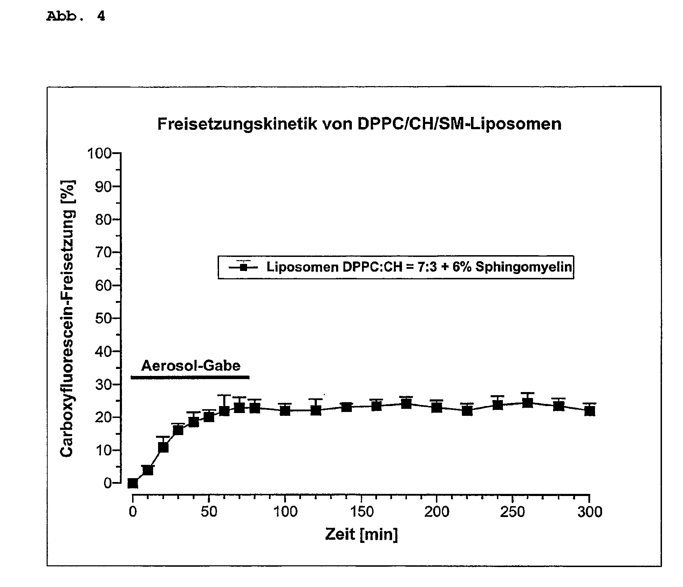

**Abb. 4 Freisetzungskinetik von DPPC/Chol/SM-Liposomen**

Liposomen der Zusammensetzung DPPC:CHOL = 7:3 + 6% Sphingomyelin mit Carboxyfluorescein als eingeschlossenem Modell-Wirkstoff werden über einen Zeitraum von 30 Minuten als Aerosol in eine isoliert ventilierte und perfundierte Kaninchenlunge eingebracht. Das aus den Liposomen freigesetzte und im Perfusionsmedium nachgewiesene Carboxyfluorescein ist in Prozent des gesamten initial in den Liposomen eingeschlossenen Carboxyfluoresceins angegeben. Dargestellt sind Mittelwerte mit Standardabweichung aus n = 3 Versuchen.

DPPC = Dipalmitoylphosphatidylcholin, Chol = Cholesterin

SM = Sphingomyelin

**Bezugszeichenliste**

> **(1) : Düsenvernebler**
> **(2) : Vorratsbeutel**
> **(3) : Ventile**
> **(4) : flexibler Ballon**
> **(5) : Glasgefäß**
> **(6) : Beatmungspumpe**
> **(7): Lunge**

**Patentansprüche**

1. Verwendung einer Liposomalen Formulierung zur Verabreichung mittels eines Verneblers, wobei die liposomale Formulierung umfasst: A) Liposomen, ausgewählt aus einer der folgenden Formulierungen: a) Liposomen umfassend i) DPPC-Phospholipide, ii) Cholesterin und iii) DMPC-phospholipide in einem molaren Verhältnis von 7:4:1 bis 7:4:4; b) Liposomen umfassend i) DPPC-Phospholipide, 11) Cholesterin und iii) polyethylenglykol in einem molaren Verhältnis von 7:3:0,15 bis 7:3:0,6; und c) Liposomen umfassend i) DPPC-Phospholipide und ii) Cholesterin in einem molaren Verhältnis von 7:3 sowie iii) Sphingomyelin-Phospholipide, die 2% bis 8% der Masse der Liposomen bilden; und B) Wirk- und/oder Farbstoff verkapselt in den Liposomen.

2. Verwendung entsprechend Anspruch 1, **dadurch gekennzeichnet, dass** die Liposomen DPPC-Phospholipide, Cholesterin und DMPC-Phospholipide in einem molaren Verhältnis von 7:4:1 bis 7:4:4 umfassen.

3. Verwendung entsprechend Anspruch 1, **dadurch gekennzeichnet, dass** die Liposomen DPPC-Phospholipide, Chollostorin und Polyethylenglykol in einem molaren Verhältnis von 7:3:0,15 bis 7:3:0,6 umfassen.

4. Verwendung entsprechend Anspruch 1, **dadurch gekennzeichnet, dass** die Liposomen DPPC-Phospholipide und Cholesterin in einem molaren Verhältnis von 7:3 umfassen sowie Sphingomyelin-Phospholipide, die 2 % bis 8 % der Masse der Liposomen bilden.

5. Verwendung entsprechend einem der Ansprüche 1 bis 5, **dadurch gekennzeichnet, dass** der Vernebler ein Düsen-Vernebler ist.

6. Verwendung entsprechend einem der Ansprüche 1 bis 5, **dadurch gekennzeichnet, dass** der Vernebler ein Ultraschallvernebler ist.

7. Verwendung entsprechend einem der Ansprüche 1 bis 5, **dadurch gekennzeichnet, dass** bei der Verabreichung 50-80% der Liposomen intakt bleiben.

8. Verwendung entsprechend einem der Ansprüche 1 bis 7, **dadurch gekennzeichnet, dass** der Wirkstoff in den Liposomen verkapselt ist.

**Claims**

1. Use of a liposomal formulation to be administered by an atomizer, with such liposomal formulation comprising: A) liposomes chosen from one of the following formulations: a) liposomes comprising i) DPPC-phospholipids, ii) cholesterol, and iii) DMPC-phospholipids at a molar ratio from 7:4:1 to 7:4:4; b) liposomes comprising i) DPPC-phospholipids, ii) cholesterol, and iii) polyethylene glycol at a molar ratio from 7:3:0,15 to 7:3:0,6, and c) liposomes comprising i) DPPC-phospholipids and ii) cholesterol at a molar ratio of 7:3, as well as iii) sphingomyelin phospholipids that form 2% to 8% of the liposomes mass; and B) active substance and/or colourant encapsulated in the liposomes.

2. Use according to Claim 1, **characterized in that** the liposomes comprise DPPC-phospholipids, cholesterol, and DMPC-phospholipids at a molar ratio from 7:4:1 to 7:4:4.

3. Use according to Claim 1, **characterized in that** the liposomes comprise DPPC-phospholipids, cholesterol, and polyethylene glycol at a molar ratio from 7:3:0,15 to 7:3:0,6.

4. Use according to Claim 1, **characterized in that** the liposomes comprise DPPC-phospholipids and cholesterol at a molar ratio of 7:3, as well as sphingomyelin phospholipids that form 2% to 8% of the liposomes mass.

5. Use according to one of the Claims 1 to 5, **characterized in that** the atomizer is a nozzle atomizer.

6. Use according to one of the Claims 1 to 5, **characterized in that** the atomizer is an ultrasonic atomizer.

7. Use according to one of the Claims 1 to 5, **characterized in that**, when being administered, 50-80% of the liposomes remain intact.

8. Use according to one of the Claims 1 to 7, **characterized in that** the active substance is encapsulated in the liposomes.

## Revendications

1. Emploi d'une formulation lipidique à être administrée par atomiseur, cette formulation lipidique comprenant : A) des liposomes choisis d'une des formulations suivantes : a) liposomes comprenant i) DPPC-phospholipides, ii) cholestérol, et iii) DMPC-phospholipides d'un rapport molaire de 7 :4 :1 à 7 :4 :4 ; b) des liposomes comprenant i) DPPC-phospholipides, ii) cholestérol, et polyéthylène-glycol d'un rapport molaire de 7 :3 :0,15 à 7 :3 :0,6, et c) des liposomes comprenant i) DPPC-phospholipides, et ii) cholestérine d'un rapport molaire de 7 :3, ainsi que iii) des phospholipides sphingo-myélines formant de 2% à 8% de la masse des liposomes ; et B) de la matière active et/ou colorante encapsulée dans les liposomes.

2. Emploi selon la Revendication 1, **caractérisé en ce que** les liposomes comprennent des DPPC-phospholipides, du cholestérol, et des DMPC-phospholipides d'un rapport molaire de 7 :4 :1 à 7 :4 :4.

3. Emploi selon la Revendication 1, **caractérisé en ce que** les liposomes comprennent des DPPC-phospholipides, du cholestérol, et du polyéthylène-glycol d'un rapport molaire de 7 :3 :0,15 à 7 :3 :0,6.

4. Emploi selon la Revendication 1, **caractérisé en ce que** les liposomes comprennent des DPPC-phospholipides et du Cholestérol d'un rapport molaire de 7 :3, ainsi que des phospholipides sphingomyélines qui forment de 2% à 8% de la masse des liposomes.

5. Emploi selon une des Revendications 1 à 5, **caractérisé en ce que** l'atomiseur est un atomiseur à buse.

6. Emploi selon une des Revendications 1 à 5, **caractérisé en ce que** l'atomiseur est un atomiseur à ultrasons.

7. Emploi selon une des Revendications 1 à 5, **caractérisé en ce que** pendant l'administration 50-60% des liposomes restent intactes.

8. Emploi selon une des Revendications 1 à 7, **caractérisé en ce que** la matière active est encapsulée dans les liposomes.

**Abb. 1**

Abb. 2

**Freisetzungskinetik von DPPC/CHOL/DMPC-Liposomen**

Abb. 3

**Freisetzungskinetik von DPPC/CH/PEG-Liposomen**

Abb. 4

## Freisetzungskinetik von DPPC/CH/SM-Liposomen

Aerosol-Gabe

—■— Liposomen DPPC:CH = 7:3 + 6% Sphingomyelin

Carboxyfluorescein-Freisetzung [%]

Zeit [min]

**EP 1 490 026 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Liposomes: a practical approach. Oxford University Press, 1994
- **Seeger et al.** *J Appl Physiol,* 1986, vol. 61, 1781-1789
- **Seeger et al.** Oxygen Radicals in Biological Systems. Academic Press, 1994, vol. 233, 549-584